# EUROPÄISCHE PATENTSCHRIFT

(11) **EP 3 174 505 B1**
(45) Veröffentlichungstag und Bekanntmachung des Hinweises auf die Patenterteilung: **12.09.2018**
(21) Anmeldenummer: 15757142.3
(22) Anmeldetag: 24.07.2015
(51) Int. Cl.: A61F 2/66, A61F 2/50, A61F 2/68, A61F 2/76

(54) **PROTHESENFUSS, SYSTEM AUS PROTHESENFUSS UND EINEM SCHUH SOWIE VERFAHREN ZUM ANPASSEN DER ABSATZHÖHE EINES PROTHESENFUSSES**
PROSTHETIC FOOT, SYSTEM COMPRISING A PROSTHETIC FOOT AND A SHOE AND METHOD OF ADJUSTING THE HEEL HEIGHT OF A PROSTHETIC FOOT
PROTHÈSE DE PIED, SYSTÈME COMPRENANT DE PROTHÈSE DE PIED ET DE CHAUSSURE ET MÉTHODE POUR RÉAJUSTER L'HAUTEUR DE TALON D'UNE PROTHÈSE DE PIED

(30) Priorität: 28.07.2014 DE 102014010938
(43) Veröffentlichungstag der Anmeldung: 07.06.2017
(73) Patentinhaber: Otto Bock HealthCare GmbH, 37115 Duderstadt (DE)
(72) Erfinder: MEYER, Hermann, 37073 Göttingen (DE); KALTENBORN, Sven, 37115 Duderstadt (DE); ALTHAUS, Steffen, 37339 Breitenworbis (DE)
(74) Vertreter: Lins, Martina
(86) Internationale Anmeldenummer: PCT/EP2015/067065
(87) Internationale Veröffentlichungsnummer: WO 2016/016147

(56) Entgegenhaltungen:
- EP-A2- 2 649 968
- DE-T2- 60 309 685
- US-A1- 2005 197 717
- US-B2- 7 520 904

## Beschreibung

Die Erfindung betrifft einen Prothesenfuß mit einem Fußteil, einem proximalen Anschlussmittel, das schwenkbar mit dem Fußteil verbunden ist und eine Verstelleinrichtung, mit der das Fußteil relativ zu dem Anschlussmittel verstellbar ist. Die Erfindung betrifft auch ein System aus einem solchen Prothesenfuß und einem Schuh sowie ein Verfahren zum Anpassen der Absatzhöhe eines Prothesenfußes an einen Schuh, dem eine Absatzhöhenmarkierung zugeordnet ist.

Bei der prothetischen Versorgung unterer Extremitäten ist es notwendig, einen für den Patienten angepassten Aufbau bereitzustellen. Unter Aufbau versteht man die Zuordnung und Ausrichtung sämtlicher Prothesenkomponenten zueinander. Bei Patienten mit einem Prothesenkniegelenk ist es notwendig, die Orientierung und Positionierung des Prothesenkniegelenkes und seiner Schwenkachse präzise auszurichten und die Länge des Unterschenkelrohrs sowie den geeigneten Prothesenfuß auszuwählen und zu montieren. Je nach Patient ist es möglich, einen dynamischen oder einen sicheren Aufbau zu wählen. Je weiter in der Standphase der resultierende Kraftvektor vor der Knieachse liegt, desto sicherer ist der Aufbau, je näher der resultierende Kraftvektor zur Schwenkachse des Prothesenkniegelenkes verschoben wird, desto dynamischer ist der Aufbau. Ist der Aufbau sicher, verringert sich die Gefahr eines ungewollten Einbeugens des Prothesenkniegelenkes, allerdings wird beim Gehen ein vergleichsweise hoher Energieaufwand notwendig. Ein dynamischer Aufbau erleichtert das Einbeugen des Prothesenkniegelenkes in der Standphase sowohl beim Fersenstoß als auch am Ende der Standphase sowie in der Schwungphase. Für das Gehen benötigt der Nutzer einen vergleichsweise geringeren Kraftaufwand, allerdings besteht die Neigung, dass das Prothesenkniegelenk leichter einbeugt, was in einigen Situationen unerwünscht sein mag.

Neben dem Aufbau des Prothesenkniegelenkes spielt der Prothesenfuß eine nicht zu unterschätzende Rolle, da über den Prothesenfuß der Kontakt zum Untergrund hergestellt wird. Der Prothesenfuß kann unterschiedlichste Ausgestaltungen aufweisen, von einem einfachen Holzklotz mit einer Kunststoffumhüllung bis zu einem hydraulisch gesteuerten, angetriebenen Prothesenfuß sind vielfältige Möglichkeiten vorhanden. Die üblichen Prothesenfüße können in Schuhen getragen werden und haben daher eine der normalen Fußform angenäherte äußere Erscheinung. Sportfüße werden in der Regel nicht in Schuhen getragen. Ein Problem bei Nutzern von Prothesen unterer Extremitäten besteht darin, dass bei einem Schuhwechsel eine Anpassung des Prothesenfußes an eine abweichende Absatzhöhe im Vergleich zu der Standardeinstellung oder der ursprünglichen Einstellung erfolgen muss. Ein Orthopädietechniker stellt bislang den optimalen Aufbau einer Prothese für einen Standard-Absatzhöhenwert ein, wird eine andere Absatzhöhe bei einem anderen Schuh gewählt oder wird ein anderes Schuhmodell mit beispielsweise einer anderen Sprengung und einer anderen Absatzhöhe verwendet, kann es notwendig sein, die Neigung des Prothesenfußes relativ zu dem Unterschenkelrohr zu verändern, um eine Absatzhöhenanpassung vorzunehmen. Anderenfalls verändert sich der gesamte Aufbau der Prothese. Eine Absatzhöheneinstellung kann auch dann notwendig werden, wenn geometrisch gleiche Schuhe verwendet werden. Aufgrund unterschiedlicher Sohlensteifigkeiten kann es notwendig sein, dass eine andere Absatzhöhe eingestellt werden muss. Ein weicherer Hacken kann eine abweichende Absatzhöhe erfordern als ein harter Hacken.

Aus dem Stand der Technik sind Prothesenfüße bekannt, die über eine hydraulische Verstelleinrichtung verfügen, so dass ein Orthopädietechniker eine Anpassung des Prothesenfußes an geänderte Absatzhöhen vornehmen kann. Nachteilig an diesen Lösungen ist, dass für jeden Schuhwechsel im Prinzip der Orthopädietechniker aufgesucht werden müsste, mit dem Ziel, einen statisch und dynamisch richtigen Aufbau zu gewährleisten. Darüber hinaus ist eine Reproduzierbarkeit der Einstellergebnisse nicht gegeben.

Von der Firma Freedom Innovations wird ein Prothesenfuß unter der Bezeichnung "Runway" angeboten. Die Einstellung erfolgt wahlweise über eine Druckknopfverstellung oder mit einem Innensechskantschlüssel.

Die Firma Streifeneder vertreibt einen Prothesenfußeinsatz mit integrierter Absatzhöhenverstellung unter der Bezeichnung "Accent", mit dem eine Höhenverstellung von bis zu 51 mm erreichbar ist.

Die US 7 520 904 B2 betrifft eine Fußprothese mit einem einstellbaren Sprunggelenk, über das der Winkel des Fußteils relativ zu einem Befestigungselement, das an einem Unterschenkelschaft oder einem Unterschenkelrohr festlegbar ist, eingestellt werden kann. Das Befestigungselement ist auf einem bogenförmigen Verschiebungsweg festgelegt und ermöglicht durch Entlanggleiten in dem Verschiebungsweg eine Anpassung an unterschiedliche Absatzhöhen. Ein Niveausensor kann ein Signal an einen Motor schicken, um das Sprunggelenk automatisch einzustellen, damit der Fuß ausgerichtet wird.

Die EP 2 649 968 A2 betrifft ein Verfahren zur Steuerung eines orthopädisches Fußteils und ein Fußteil, das ein Unterschenkel-Anschlussteil, ein als Knöchelgelenk wirkendes Drehgelenk, mit dem ein Fersenteil in Richtung Dorsalflexion und Plantarflexion drehbar mit dem Anschlussteil verbunden ist, eine die Drehbewegung um das Drehgelenk beeinflussende Dämpfungsanordnung, eine Sensoranordnung zur Erkennung von Aktionszuständen des orthopädischen Fußteils und eine mit der Sensoranordnung verbundene Steuerung aufweist, die die Dämpfungsanordnung steuert. Ein zu dem an dem Knöchelgelenk auftretenden Drehmoment proportionaler Wert, der Knöchelwinkel zwischen dem Anschlussteil und dem Fersenteil und der Absolutwinkel des Fersenteils bezogen auf die Lotrechte werden bestimmt. In Abhängigkeit von diesen gemessenen Werten werden das Abrollen des Fußes in der Standphase, die Stellung des Fersenteils in der Schwungphase und die Stellung und Bewegbarkeit des Fersenteils während des Stehens mittels der Dämpfungsanordnung gesteuert. Die Beeinflussung von Parametern eines Gangzyklus erfolgt durch für diesen Gangzyklus bereits gemessene Sensorsignale.

Die US 2005/0197717 A1 betrifft ein System und ein Verfahren zur Bewegungssteuerung eines Prothesenfußes. An einem Fußteil ist gelenkig eine Befestigungseinrichtung zur Befestigung des Prothesenfußes an einem Unterschenkelrohr oder Unterschenkelschaft vorgesehen. Dem Knöchelgelenk ist ein Aktuator zugeordnet, der über einen Rechner angesteuert ist. Ein Sensormodul versorgt den Rechner mit Daten über Belastungen, Beschleunigungen, Winkel oder myoelektrische Daten. Ein Interfacemodul kann dem Rechner zugeordnet sein, um über verschiedene Knöpfe oder LEDs Informationen zu erhalten oder einzugeben. Über den Aktuator wird der Winkel zwischen dem Befestigungsteil und dem Fußteil eingestellt, bevorzugt auf der Basis von Informationen über den Bewegungsfortschritt.

Die DE 603 09 685 T2 betrifft ein elektronisch gesteuertes Prothesensystem, das die natürliche menschliche Fortbewegung und die menschliche Biomechanik über Sensorfeedback, Zeitfeedback, eine elektronisch gesteuerte Dämpfungsgelenkeinheit und ein Mikroprozessor-Steuersystem simuliert. Zur Erzielung einer verbesserten Ästhetik und Funktion wird das Dämpfungssystem gesteuert und reguliert, wobei ein magnetorheologisches Fluid genutzt wird. Das Dämpfersystem ist zwischen einem Fußteil und einer Befestigungseinrichtung angeordnet und dämpft eine Schwenkbewegung der Befestigungseinrichtung relativ zu dem Fußteil. Über Aktivierung oder Deaktivierung von Magneten wird ein innerhalb der Dämpfereinrichtung vorhandenes magnetorheologisches Fluid in seiner Viskosität verändert. Ein Winkelsensor ist vorgesehen, um den relativen Winkel des Fußteils zur unteren Extremität des Benutzers zu ermitteln.

Aufgabe der vorliegenden Erfindung ist es, einen Prothesenfuß, ein System aus einem Prothesenfuß und einem Schuh sowie ein Verfahren zum Anpassen einer Absatzhöhe eines Prothesenfußes an einen Schuh bereitzustellen, mit denen ein Prothesenfußnutzer den Prothesenfuß an den jeweiligen Schuh anpassen kann, wobei die Einstellung reproduzierbar durchzuführen ist, um dadurch den optimierten Prothesenaufbau beizubehalten.

Erfindungsgemäß wird diese Aufgabe durch einen Prothesenfuß mit den Merkmalen des Hauptanspruches, ein System aus einem Prothesenfuß und einem Schuh mit den Merkmalen des nebengeordneten Anspruchs sowie solch ein Verfahren mit dem nebengeordneten Verfahrensanspruch gelöst. Vorteilhafte Ausgestaltungen und Weiterbildungen der Erfindung sind in den Unteransprüchen, der Beschreibung und den Figuren offenbart. Mit der Erfindung ist es möglich, eine verbesserte prothetische Versorgung bereitzustellen. Bei Patienten mit transtibialer Amputation führen falsche Absatzhöheneinstellungen zu Schädigungen von Kapseln und Bändern sowohl beim versorgten als auch beim unversorgten Bein, bei transfemoralen Amputationen kann eine nicht optimierte Absatzhöheneinstellung und einer Veränderung des Prothesenaufbaus zu Dehnungen in dem Kapsel- und Bandapparat des Knies des unversorgten Beines sowie zu Überlastungen einzelner Muskeln führen. Mit der Erfindung werden diese Nacheile vermindert oder verhindert.

Der erfindungsgemäße Prothesenfuß mit einem Fußteil, einem proximalen Anschlussmittel, das schwenkbar mit dem Fußteil verbunden ist und einer Verstelleinrichtung, mit der das Fußteil relativ zu dem Anschlussmittel verstellbar ist, sieht vor, dass der Verstelleinrichtung zumindest ein Positionssender zugeordnet ist, der mit einem Signalerzeugungselement gekoppelt ist, das in Abhängigkeit von dem Signal des zumindest einen Positionssensors ein Signal über das Erreichen einer Position des Fußteils ausgibt. Zur Einstellung der Absatzhöhe ist es notwendig, die Neigung des Fußteils relativ zu dem Anschlussmittel zu verändern. Ausgehend von einer durch den Orthopädietechniker eingestellten Absatzhöhe, wird der Prothesenaufbau von einem Orthopädietechniker optimiert. Wird ausgehend von einer Ausgangsstellung das Fußteil bei einer veränderten Absatzhöhe in Plantarrichtung flektiert, also die Fußspitze in Richtung auf den Boden verschwenkt, so dass die Ferse relativ angehoben wird, detektiert der Positionssensor die Winkelstellung des Fußteils relativ zu dem proximalen Anschlussmittel, mit dem der Prothesenfuß an dem Unterschenkelrohr befestigt wird. Das Signalerzeugungselement kann als ein aktives Signalerzeugungselement ausgebildet sein und in Abhängigkeit von dem Signal, das von dem Positionssensor an das Signalerzeugungselement übermittelt wird, beispielsweise nach Auswertung in einer Steuereinheit, ein optisches, akustisches oder taktiles Signal ausgeben, so dass der Prothesennutzer weiß, welche Stufe der Absatzhöheneinstellung gegenwärtig vorliegt. Die Ausgabe kann akustisch erfolgen, beispielsweise durch eine Sprachausgabe, mit der die jeweils erreichte Absatzhöhenabstufung angezeigt wird. Ebenso ist es möglich, dass nach Erreichen der jeweils nächsten Absatzhöhenstufe ein Signalton ausgegeben wird, wobei den jeweiligen Absatzhöhenstufen unterschiedliche Signaltöne zugeordnet werden können. Auch ist es möglich, dass unterschiedliche Tonhöhen, Signalmuster oder auch Signalfolgen der jeweiligen Absatzstufe zugeordnet ist. Liegen beispielsweise sechs Absatzhöhenstufen vor, kann jede mit einer entsprechenden Anzahl an Tönen charakterisiert werden, die dritte Stufe mit drei aufeinanderfolgenden Signaltönen, die vierte Stufe mit vier und so weiter. Neben einer akustischen Signalausgabe ist es möglich, eine taktile Signalausgabe vorzusehen, beispielsweise durch Vibrationen, Vibrationsmuster oder dergleichen. Weiterhin ist es alternativ oder ergänzend möglich, ein optisches Signal auszugeben, beispielsweise ein Lichtsignal, Lichtsignalmuster oder gegebenenfalls eine Anzeige der gerade vorhandenen Absatzhöhenstufe. Sämtliche Signale können für jede Stufe alternativ oder kumulativ eingesetzt werden. Auch ist es möglich, für unterschiedliche Absatzhöhenstufen unterschiedliche Signalarten auszugeben, beispielsweise können bestimmte Absatzhöhenstufen nur akustisch, andere nur optisch und wiederum andere nur haptisch angezeigt werden. Eine optische Anzeige kann beispielsweise auch über unterschiedliche Farbsignale erfolgen. Durch die Signalausgabe ist es nicht mehr notwendig, für jede Absatzhöhenänderung den Orthopädietechniker aufzusuchen, vielmehr ist es ausreichend, für verschiedene Absatzhöhen die jeweiligen Absatzhöhenstufen den Signalen zuzuordnen, so dass nach dem einmaligen Einstellen des Prothesenaufbaus und gegebenenfalls der Zuordnung unterschiedlicher Absätze mit unterschiedlichen Absatzhöhen zu den jeweiligen Signalen der Patient eigenständig bei Kenntnis der jeweiligen Absatzhöhe des jeweiligen Schuhmodels die Anpassung des Prothesenfußes an die jeweilige Absatzhöhe vornehmen kann. Neben den beschriebenen aktiven Signalausgabeelementen oder Ausgabeeinrichtungen ist es möglich und vorgesehen, dass passive Signalerzeugungselemente vorgesehen sind, die keine gesonderte Energiequelle zur Signalerzeugung benötigen. Beispielsweise kann über eine Rasterung bei der Verstellung ein taktiles Signal über sogenannte Rattermarken ausgegeben werden, so dass der Prothesennutzer oder Orthopädietechniker weiß, um wie viele Rastschritte oder Absatzhöheneinstellstufen das Fußteil gegenüber dem Anschlussmittel verschwenkt wurde. Neben einer passiven und taktilen Ausgabeeinrichtung, die auch eine akustische Komponente aufweist, ist es auch möglich, dass eine optische Anzeige der Absatzhöheneinstellung vorliegt, beispielsweise über eine farbliche Markierung in einem Sichtfenster, die Anzeige einer bestimmten Raststufe durch alphanumerische Zeichen oder andere Symbole.

Die Zuordnung der Signale sowie die jeweiligen Absatzhöheneinstellung kann auch chronologisch erfolgen. Der Orthopädietechniker und/oder der Patient können eine beliebige Absatzhöhe verwenden, um den Prothesenaufbau korrekt einzustellen. Dieser Absatzhöhe wird dann in Abhängigkeit von dem Zeitpunkt der Einstellung ein entsprechendes Signal zugeordnet. Ist es die erste eingestellte Absatzhöhe, erfolgt beispielsweise ein einmaliger Signalton oder ein einmaliges Lichtsignal oder ein einmaliges taktiles Signal. Eine zweite Absatzhöhe, die von der ersten Absatzhöhe verschieden ist, erhält chronologisch ein zweites Signal, beispielsweise zwei Signaltöne, Lichtsignale oder taktile Signalausgaben. Die dritte Speicherung einer Absatzhöhe erhält ein drittes Signal usw., wobei die Absatzhöhen beliebig sein können, die erste Absatzhöhe kann einen mittleren Absatz repräsentieren, die zweite Speicherung einen hohen Absatz und die dritte einen sehr flachen Absatz.

Die Ausgabe des Absatzhöhensignals durch das Signalerzeugungselement kann absolut erfolgen, so dass unabhängig von einer Ausgangsstellung die jeweilige eingenommene Position angesagt wird. Alternativ dazu kann die Signalausgabe stets ausgehend von einer Standardausgangsstellung erfolgen, so dass beispielsweise nach der Aktivierung eines Einstellmodus jeweils nur die Anzahl der gerade vorgenommenen Absatzhöhenverstellstufen ausgegeben wird.

Die Verstelleinrichtung kann Rastelemente aufweisen, über die die Verstellung des Fußteils relativ zu dem Anschlussmittel in diskreten Schritten erfolgt. Die Rasterung ist hinreichend fein zu wählen, so dass ein möglichst präzise Einstellung an die jeweiligen Absatzhöhen erfolgen kann, andererseits ist die Rasterung so grob zu wählen, dass nicht zu viele Signale ausgegeben werden, die bei der Einstellung verwirren könnten. Es hat sich als ausreichend herausgestellt, dass 16 bis 20 Absatzhöhenschritte für eine effektive Absatzhöhenänderung zwischen 0 cm und 5 cm für eine ausreichend feine Absatzhöheneinstellung bei den üblichen Schuhmodellen ausreichen.

Der Positionssensor kann als Lagesensor, Relativwinkelsensor, Inertialwinkelsensor, mechanischer Sensor und/oder Schalter ausgebildet sein. Bei einem Relativwinkelsensor wird die relative Position zwischen dem Anschlussmittel und dem Fußteil erfasst, beispielsweise über in diskreten Schritten angeordnete Magnete, über Induktion, einen Hall-Sensor oder dergleichen. Ebenso ist es möglich, verschiedene Marker in diskreten Winkelabständen zueinander anzuordnen, die von einem Detektor erkannt und von dem ein entsprechendes Signal entweder direkt an das Signalerzeugungselement oder an eine Steuereinrichtung gesendet wird. Relativ dazu ist es möglich, an dem Fußteil einen Lagesensor oder Inertialwinkelsensor vorzusehen, über den die Absolutstellung des Fußteils relativ zu einer feststehenden Bezugsgröße, in der Regel der Gravitationsrichtung, ermittelt wird. Nach einer einmaligen Ausrichtung des Fußteils, beispielsweise in einer Stellung ohne Schuh, kann dann durch die Veränderung der Lage des Fußteils relativ zu der Gravitationsrichtung in einem Einstellmodus festgestellt werden, um wieviel Grad eine Verstellung in Plantarflexionsrichtung erfolgt, woraus sich dann in Verbindung mit der Prothesenfußlänge errechnen lässt, bei welcher Fußteilstellung ein bestimmter Absatzhöhenschritt erreicht ist. Die Einstellung der Absatzhöhe erfolgt in der Regel unter Belastung, das heißt, dass der Patient bei einem Orthopädietechniker gleichmäßig belastet steht. Dies bedeutet, dass in der Regel 50% des Körpergewichtes auf dem versorgten Bein und 50% auf dem unversorgten Bein getragen werden. Die Speicherung der jeweiligen Absatzhöheneinstellung zu dem jeweiligen Schuh oder Schuhmodell erfolgt nach einer statischen und dynamischen Anpassung des Prothesenaufbaus durch den Orthopädietechniker. Der Inertialwinkelsensor kann als Gyroskop ausgebildet sein. Auch ist es möglich, dass der Positionssensor als ein einfacher Schalter ausgebildet ist, der durch ein mechanisches Betätigungselement umgelegt wird, so dass ein Schaltkreis geöffnet oder unterbrochen wird. Je nach Öffnen oder Schließen des Schaltkreises wird ein entsprechendes Signal ausgegeben. Solche Schalter können auch separat aktiviert werden, um eine individuelle Stufung zu bewirken, ähnlich einer mechanischen Zeitschaltuhr mit manuell aktivierbaren Schaltzeitpunkten. Die Ausgestaltung als mechanischer Sensor sieht vor, dass der Positionssensor ohne elektrische oder elektronische Komponenten funktionieren kann, beispielsweise indem eine optische Anzeige oder eine akustisches Signal auf rein mechanische Art und Weise erzeugt oder verändert wird. Die Funktionsweise entspricht dabei der eines Schalters, allerdings ohne das Schließen oder Öffnen eines Kontaktes.

Der Sensoreinrichtung kann ein einstellbarer oder programmierbarer Signalgeber zugeordnet sein, so dass sowohl die Abstände zwischen den ausgegebenen Signalen oder auch die Art des jeweiligen Signals festgelegt werden kann. Auch ist es möglich, dass ein Signal erst dann und nur dann ausgegeben wird, wenn die gewünschte Verstellung erfolgt ist. Wird beispielsweise eine bestimmte Absatzhöhe detektiert oder eingestellt, kann über eine Bestätigungseinrichtung definiert werden, dass ein Signal nur dann ausgegeben wird, wenn die jeweils eingestellte Absatzhöhenposition erreicht wird. Auf diese Art und Weise ist es auch möglich, über eine automatische Absatzhöhenerkennung eine einfache Anpassung der Orientierung des Fußteils relativ zu den proximalen Anschlussmitteln zu bewirken, da nur noch auf das Vorliegen oder Nichtvorliegen eines Signals geachtet werden muss. Ist die korrekte Einstellung erreicht, wird das Signal ausgegeben oder umgekehrt unterbrochen. Dies ist für den Prothesennutzer das Zeichen, die Verstellung zu unterbrechen und die gefundene Position zu fixieren.

Der Verstelleinrichtung kann zumindest ein Empfänger oder ein Detektor von Schuhidentifikationsdaten zugeordnet sein, so dass automatisch erkannt werden kann, welches Schuhmodel bzw. welche Absatzhöhe an dem jeweiligen Prothesenfuß angeordnet ist. Über den Empfänger oder den Detektor erkennt der Prothesenfuß, in welchen Schuh er eingeführt wurde, so dass eine automatische Anpassung des Signalgebers oder eine Steuereinrichtung erfolgt, so das eine vereinfachte Anpassung aufgrund der automatischen Detektion der jeweiligen Absatzhöhe erfolgen kann. Durch den Detektor wird beispielsweise automatisch erkannt, welche Absatzhöhe vorliegt, so dass über die Steuereinrichtung festgelegt wird, dass erst bei Erreichen eines bestimmten Verstellwinkels ein entsprechendes Signal ausgegeben wird. Auf diese Weise wird automatisch und reproduzierbar die direkte Winkeleinstellung für das Fußteil vorgenommen.

Der Empfänger oder Detektor ist mit der Sensoreinrichtung und/oder dem Signalerzeugungselement gekoppelt, um den oben beschriebenen automatischen Abgleich entweder des Signals oder der Signalauslösung vornehmen zu können.

Das Signalerzeugungselement kann ebenfalls mit einem Sender gekoppelt sein, so dass das detektierte Signal des Sensors nicht unmittelbar an der Protheseneinrichtung ausgegeben werden muss, sondern auch an eine entfernt angeordnete Einrichtung übermittelt werden kann. Beispielsweise kann eine Signalübertragung an ein Mobiltelefon, ein Tablet, einen Computer oder eine andere Ausgabeeinrichtung erfolgen, so dass neben der Signalausgabe oder -anzeige, die bei Mobiltelefonen sowohl optisch, als auch akustisch und/oder taktil erfolgen kann, eine Speicherung der Werte und verwendeten Schuhe bzw. Absatzhöheneinstellungen vorgenommen werden kann. Die dadurch vorhandenen Daten können zur Optimierung der Einstellung des Prothesenaufbaus verwendet werden.

Die Verstelleinrichtung kann eine exzentrisch an dem Fußteil gelagerte Welle aufweisen, die wiederum an dem Anschlussmittel gelagert ist. Dadurch ist auf eine einfache Art und Weise die Möglichkeit gegeben, durch Verdrehung der Exzenterwelle die Verstellung der proximalen Anschlussmittel relativ zu dem Fußteil zu bewirken. Alternative Ausgestaltungen der Verstelleinrichtungen, beispielsweise über Gewindestangen, Rasteinrichtungen oder andere formschlüssige Verriegelungen, sind ebenso möglich und vorgesehen. Die Ausgestaltung über eine Exzenterwelle ermöglicht eine räumlich kleine Ausgestaltung bei ausreichend feiner Verstellung und sicherer Verriegelung. Die Exzenterwelle kann in einem Joch gelagert sein, an dem auch die Anschlussmittel verschwenkbar gelagert sind, wodurch die Möglichkeit besteht, die Exzenterwelle im Wesentlichen parallel zu der Schwenkachse und der Lagerungsachse der Anschlussmittel auszugestalten. Um die bei der Verdrehung auftretende Längsverlagerung der Exzenterwelle auszugleichen, ist die Welle vorteilhafterweise in einer Langlochführung in dem Anschlussmittel geführt. Das Langloch kann durch die Anschlussmittel hindurchgehen. Selbstverständlich ist es möglich und vorgesehen, dass die exzentrische Welle an dem Anschlussmittel gelagert ist, die mit dem Anschlussmittel gekoppelt ist. Auch kann es sein, dass die Langlochführung nicht in dem Anschlussmittel, sondern in dem Fußteil ausgebildet ist. Die Verstelleinrichtung ist somit über eine exzentrisch gelagerte Welle mit dem Anschlussmittel gekoppelt, wobei die Welle vorzugsweise in einer Langlochführung geführt ist.

Das Anschlussmittel kann eine deaktivierbare Sperreinrichtung aufweisen, die eine Verdrehung des Fußteils relativ zu dem Anschlussmittel sperrt, so dass die einmal gefundene Orientierung des Fußteils relativ zu dem Anschlussmittel beibehalten wird. Die Sperreinrichtung weist vorteilhafterweise ausrückbare Formschlusselemente auf, so dass eine Aktivierung bzw. Deaktivierung einfach möglich ist.

Das Signalerzeugungselement ist bevorzugt als aktives Signalerzeugungselement ausgebildet, um dem Nutzer ein eindeutiges und einfach aufzunehmendes Signal über die eingestellt Absatzhöhe zu liefern.

Das System aus einem oben beschriebenen Prothesenfuß mit einem Schuh sieht an dem Schuh eine auslesbare Absatzhöhenmarkierung vor, so dass entweder optisch, opto-elektronisch oder elektronisch die jeweils vorliegende Absatzhöhenstufe ausgelesen, der Steuerungseinrichtung, dem Positionssensor oder dem Signalerzeugungselement übermittelt und anschließend ein entsprechendes Signal bei Erreichen der korrekten Absatzhöheneinstellung ausgegeben werden kann. Die Absatzhöhenmarkierung kann in einem RFID-Chip, einem Transponder, einem optisch oder einem opto-elektronisch auslesbaren Code abgelegt sein. Die Absatzhöhenmarkierung kann auch den Schuh aufgeklebt, eingeprägt, eingebettet oder auf andere Art und Weise angebracht sein.

Das erfindungsgemäße Verfahren zum Anpassen der Absatzhöhe eines Prothesenfußes, wie er oben beschrieben worden ist, an einem Schuh, dem eine Absatzhöhenmarkierung zugeordnet ist, sieht vor, dass die Absatzhöhenmarkierung an eine Steuereinrichtung übermittelt wird, die jeweilige Markierung mit erfassten Positionsdaten des Positionssensors verglichen und ein Bestätigungssignal ausgegeben wird, wenn das der jeweiligen Absatzhöhenmarkierung vorab zugeordnete Positionssignal mit dem erfassten Positionssignal übereinstimmt. Die Absatzhöhenmarkierung kann näherungsabhängig von einem Detektor erfasst und das entsprechende Signal der Steuerungseinrichtung übermittelt werden, beispielsweise wenn ein Detektor oder ein Sender in eine ausreichende Nähe oder direkten Kontakt mit der Absatzhöhenmarkierung gelangt. Auch ist es möglich, über optische Detektoren, beispielsweise Scanner, einen entsprechenden Code auszulesen und der Steuerungseinrichtung zu übermitteln, so dass nach Auslesen und Verarbeiten des entsprechenden Codes eine entsprechende Signalzuordnung und Signalausgabe erfolgt.

Neben einer automatischen Erfassung und Auslesung der Absatzhöhenmarkierung und der automatischen Übermittlung an die Steuerungseinrichtung ist es grundsätzlich auch möglich, die jeweilige Absatzhöhe manuell der Steuerungseinrichtung zu übermitteln, beispielsweise durch Tasten, eine Fernbedienung oder eine Rasterung oder Bestätigung einer bestimmten Rastposition.

In einer Variante der Erfindung ist dem Prothesenfuß ein Antrieb zugeordnet, so dass das Anschlussmittel relativ zu dem Fußteil motorisch verlagert wird, wobei die Verlagerung in Abhängigkeit von der detektierten Absatzhöhe erfolgt, so dass stets eine automatische Anpassung der Stellung des Fußteils relativ zu dem Anschlussmittel beim Anlegen eines Schuhs mit einer entsprechenden Absatzhöhenmarkierung erfolgt.

Nachfolgend werden Ausführungsbeispiele der Erfindung anhand der Figuren näher erläutert. Es zeigen:
- Fig. 1 -: einen Prothesenfuß in einem Schuh;
- Fig. 2 -: einen Prothesenfußeinsatz in Seitenansicht;
- Fig. 3 -: einen Prothesenfußeinsatz nach Fig. 2 mit Fußkosmetik;
- Fig. 4 -: einen verstellten Prothesenfußeinsatz;
- Fig. 5 -: einen Prothesenfußeinsatz nach Fig. 4 mit Fußkosmetik;
- Fig. 6 -: ein Joch mit Anschlussmittel in Seitenansicht;
- Fig. 7 -: Fig. 6 in perspektivischer Ansicht;
- Fig. 8 -: eine Explosionsdarstellung der Verstelleinrichtung;
- Fig. 9 -: eine Schnittdarstellung der Verstelleinrichtung;
- Fig. 10: eine Variante der Erfindung in Seitenansicht; sowie
- Fig. 11: eine Detailansicht eines Positionssenors.

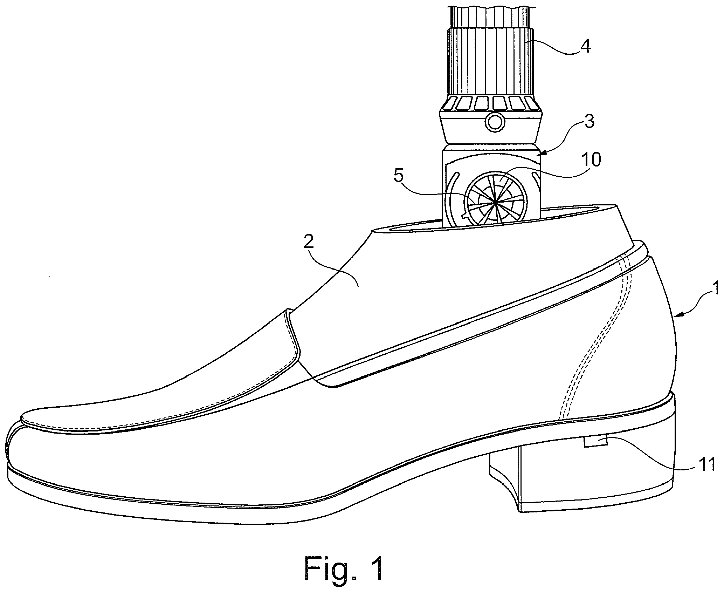

Figur 1 zeigt einen Schuh 1 mit einem darin befindlichen Prothesenfuß, der ein Fußteil 2 und proximale Anschlussmittel 3 zum Anschluss an ein Unterschenkelrohr 5 aufweist. Die proximalen Anschlussmittel 3 sind verschwenkbar an dem Fußteil 2 gelagert und weisen eine Verstellvorrichtung 5 auf, über die die Neigung des Fußteils 2 relativ zu dem Anschlussmittel 3 bzw. zum Unterschenkelrohr 4 eingestellt werden kann. Über die Neigungseinstellung des Fußteils 2 können bei einem Schuhwechsel die unterschiedlichen Absatzhöhen ausgeglichen werden. In dem Schuh 1 ist eine Absatzhöhenmarkierung 11 angeordnet, im dargestellten Ausführungsbeispiel im Bereich des Absatzes, über die es möglich ist, die jeweilige Absatzhöhe zu erkennen und entweder manuell oder automatisch an die Verstelleinrichtung 3 bzw. eine in der Figur 1 nicht dargestellte Steuereinrichtung, ein Signalerzeugungselement 10 oder eine Sensoreinrichtung zu übermitteln. An der Verstelleinrichtung 5 ist ein Signalerzeugungselement in Gestalt einer Ausgabeeinrichtung für akustische, taktile und/oder optische Signale angeordnet, über die der Prothesennutzer über die jeweilige Einstellung der Absatzhöhe informiert werden kann.

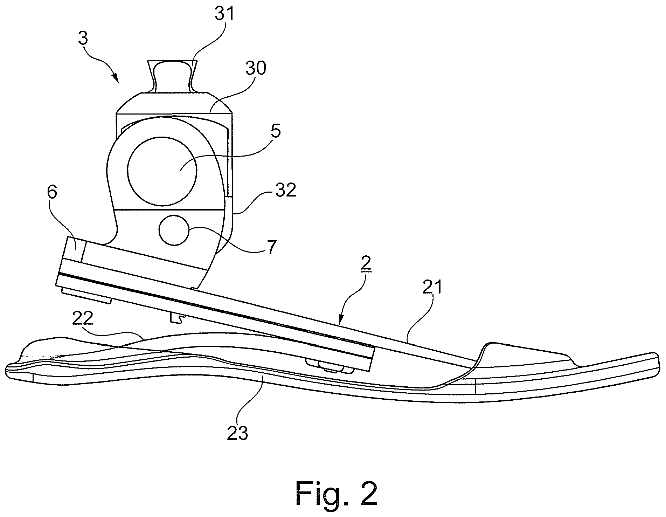

Figur 2 zeigt einen Prothesenfußeinsatz ohne eine Fußkosmetik mit einem Fußteil 2, das eine Vorderfußfeder 21, eine Fersenfeder 22 und eine Basisfeder 23 aufweist. An dem hinteren Ende der Vorderfußfeder 21 ist ein Joch 6 angeordnet, das sich in proximaler Richtung erstreckt und zur Aufnahme des proximalen Anschlussmittels 3 dient. Das Joch 6 ist fest mit der Vorfußfeder 21 verschraubt. Das Anschlussmittel 3 weist einen Grundkörper 30 mit einem Pyramidenadapter 31 auf. Ein Unterteil 32 ist an dem Grundkörper 30 befestigt und dient als Aufnahme für eine Schwenkachse 7, die quer durch das Joch 6 hindurchgeht. Das Anschlussmittel 3 ist zwischen den beiden Schenkeln des Joches 6 angeordnet und um die Schwenkachse 7 verschwenkbar gelagert. Weiterhin ist in dem Joch 6 proximal zur Schwenkachse 7 eine Verstelleinrichtung 5 gelagert, deren Aufbau später erläutert wird. Über die Verstelleinrichtung 5 ist es möglich, die Winkelstellung des Fußteils 2 relativ zu dem Anschlussmittel 3 zu verändern. Durch unterschiedliche Neigungen des Anschlussmittels 3 relativ zu dem Fußteil 2 ist es möglich, unterschiedliche Absatzhöhen für nicht dargestellte Schuhe auszugleichen.

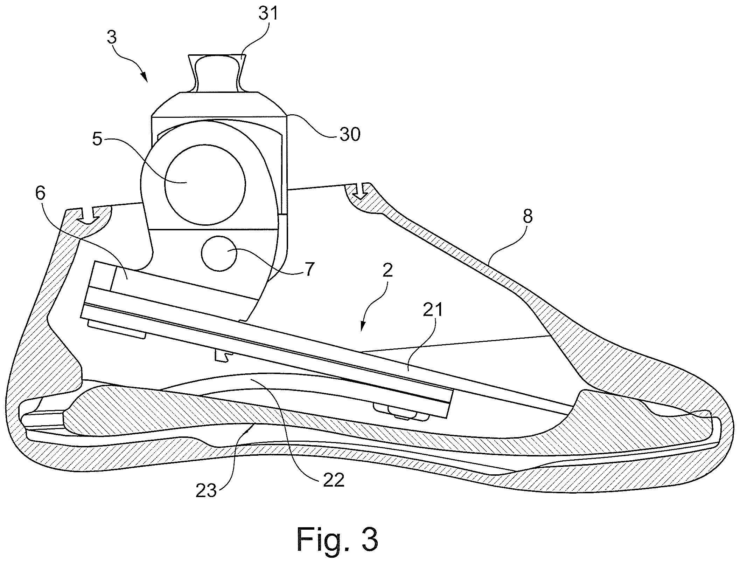

Figur 3 zeigt den Prothesenfußeinsatz gemäß Figur 2 mit einer Fußkosmetik 8, in die der Prothesenfußeinsatz zusammen mit dem Joch 6 und Teilen des Anschlussmittels 3 eingeführt ist. Der Prothesenfußeinsatz zusammen mit der Fußkosmetik bildet den Prothesenfuß, der in einen Schuh eingeführt wird. Die Fußkosmetik 8 bildet den distalen Abschluss des Prothesenfußes und trägt aufgrund der elastischen Eigenschaften zum Funktionsverhalten des Prothesenfußes bei. In dem dargestellten Ausführungsbeispiel ist die Ausrichtung des Anschlussmittels 3 relativ zu dem Fußteil 2 so gewählt, dass eine relativ flache Absatzhöhe vorliegt, die Sohle oder Unterseite der Fußkosmetik 3 ist an ihren Auflagepunkten auf einem nahezu waagerechten Niveau.

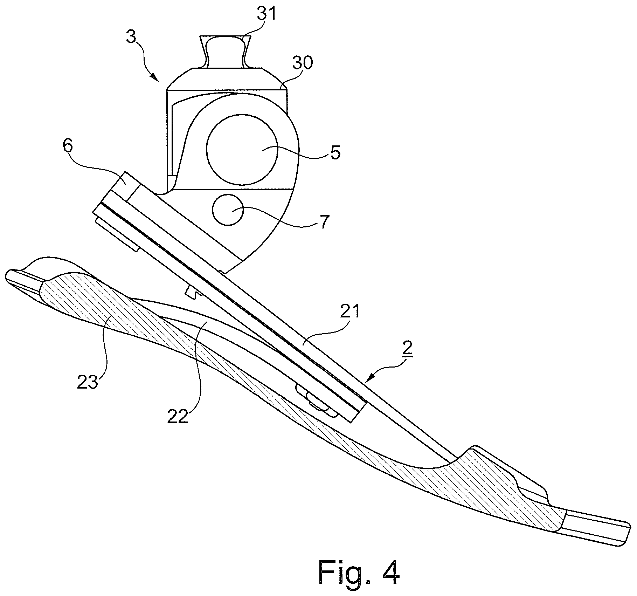

Figur 4 zeigt eine Variante der Figur 2 in einer maximal angewinkelten Stellung, das heißt, dass das Fußteil 2 eine maximale Plantarflexion aufweist und das Anschlussmittel 3 um die Schwenkachse 7 maximal entgegen der Uhrzeigerrichtung relativ zu dem Joch 6 verdreht worden ist. Die Fußspitze steht wesentlich tiefer als die Ferse. Eine solche Fußstellung ist notwendig, wenn auf der anderen bzw. unversorgten Seite des Patienten ein Schuh mit einem hohen Absatz getragen wird. Würde die Fußstellung gemäß Figur 2 bei einer vergrößerten Absatzhöhe genutzt werden, würde dies zu einem nicht handhabbaren Abrollverhalten und einem sehr unphysiologischen Gangbild führen.

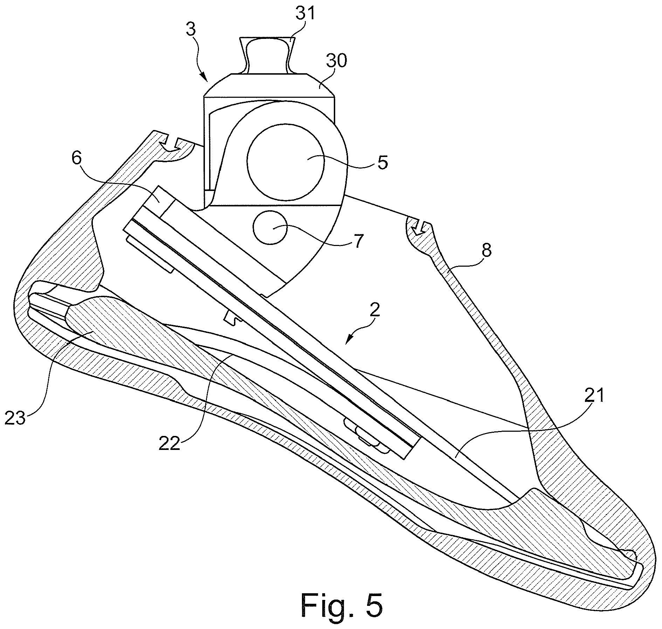

In der Figur 5 ist die Fußstellung gemäß Figur 4 mit einer Fußkosmetik gezeigt.

Um die Fußstellungen gemäß der Figuren 3 und 5 zusammen mit einem von einem Orthopädietechniker eingestellten Prothesenaufbau nutzen zu können, ist es notwendig, die Absatzhöhe einstellbar zu gestalten. Hierzu ist eine Verstelleinrichtung 5 im Bereich des Anschlussmittels 3 an der Lagerung vorgesehen.

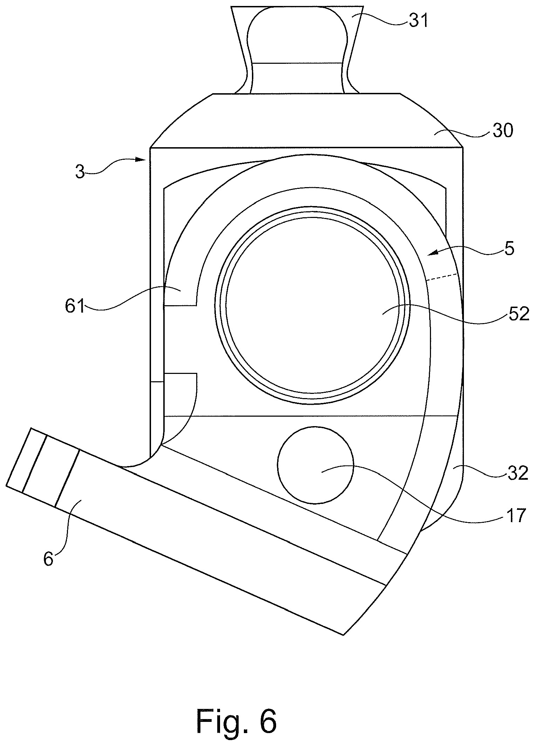

Figur 6 zeigt die Verbindung des oberen Anschlussmittels 3 mit dem Pyramidenadapter 31, dem Grundkörper 30 und dem Unterteil 32 an dem Joch 6 in einer Einzeldarstellung. Die proximale Schwenkachse 7 ist ebenso zu erkennen wie die distal dazu angeordnete Verstelleinrichtung 5. Figur 6 zeigt eine Seitenansicht auf einen Träger 61 oder Jochschenkel mit einer kreisförmigen Ausnehmung, in der eine Lagerscheibe 52 gelagert ist. Die Funktionsweise der Lagerscheibe 52 und der Verstelleinrichtung 5 wird nachfolgend erläutert.

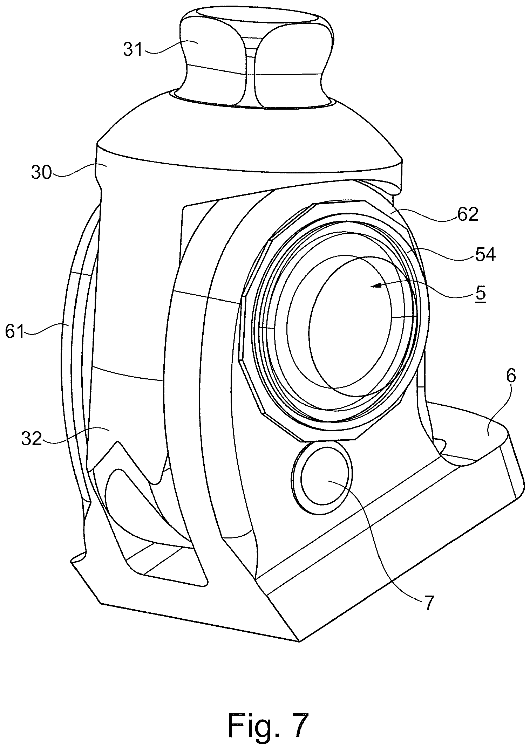

Figur 7 zeigt die Anordnung gemäß Figur 6 in einer perspektivischen Ansicht. Es ist zu erkennen, dass das Unterteil 3 an dem Grundkörper 30 festgelegt ist und sowohl der Grundkörper 30 als auch das Unterteil zwischen zwei Trägern 61, 62 oder Jochschenkeln des Joches 6 angeordnet ist. Die Verstelleinrichtung 5 weist auf der Seite des zweiten Trägers 62 eine Abdeckkappe 54 auf, um verschiedene Komponenten der Verstelleinrichtung 5 zu schützen. Im dargestellten Ausführungsbeispiel ist das Anschlussmittel 3 als ein Grundkörper 30 mit einem direkt daran befestigten Pyramidenadapter 31 ausgestaltet. Neben einer solchen Ausgestaltung ist es auch möglich, zwischen dem Pyramidenadapter 31 und dem Joch 6 oder dem Fußteil 2 einen Adapter vorzusehen, um beispielsweise eine einfachere Längeeinstellung vorzunehmen oder auch um in dem Adapter Komponenten der Verstelleinrichtung 5 unterzubringen. So kann in dem Adapter eine Ausgabeeinrichtung, eine Steuereinrichtung, eine Eingabeeinrichtung für die Programmierung der jeweiligen Absatzhöhe und die Zuordnung zu einem Signal untergebracht sein. Die Verstellung selbst kann auch in dem Adapter durchgeführt werden, so dass nicht notwendigerweise unmittelbar an dem Grundkörper 30 der Verstelleinrichtung 3 der Pyramidenadapter 31 angeordnet, ausgebildet oder befestigt sein muss. Der Pyramidenadapter 31 kann auch über ein zwischengeschaltetes Zwischenstück oder einen Adapter entweder mit dem Grundkörper 31 oder auf andere Art und Weise schwenkbar mit dem Fußteil 2, beispielsweise über ein Joch 6 oder eine andere Art und Weise der schwenkbaren Befestigung an dem Fußteil 2 befestigt sein. Neben einer Anordnung der oberen Anschlussmittel 3 zwischen den beiden Trägern 61, 62 in dem Joch ist es auch möglich den Grundkörper 3 nur einseitig an einem einzigen Träger anzuordnen oder aber einen zentralen Träger beidseitig von Komponenten der oberen Anschlussmittel 3 einzufassen, so dass die umgekehrte Anordnung verglichen mit dem dargestellten Ausführungsbeispiel verwirklicht wird.

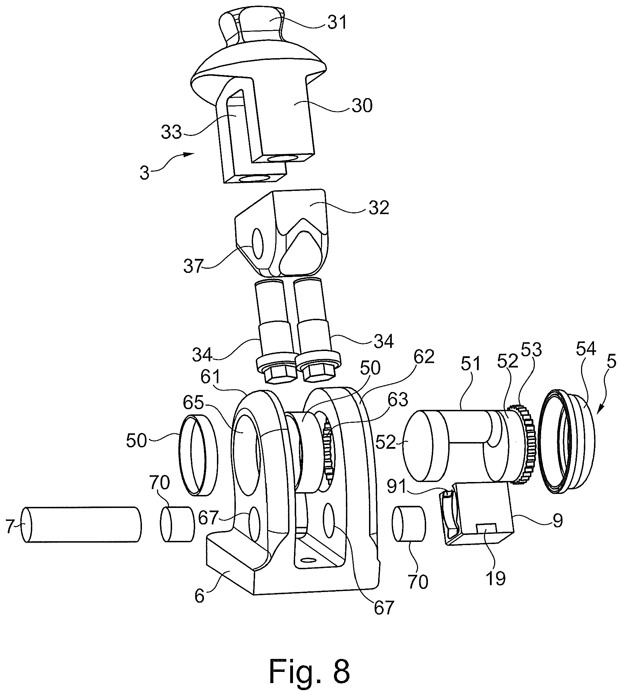

Figur 8 zeigt in einer Explosionsdarstellung das Joch 6 mit Durchgangsbohrungen 67 im distalen Bereich der Träger 61, 62 zur Aufnahme der Schwenkachse 7. Die Schwenkachse 7 ist in Lagern 70, die als Gleitlager ausgebildet sind, in den Durchgangsbohrungen 70 gelagert. Die Schwenkachse 7 durchragt im montierten Zustand sowohl die Träger 61, 62 als auch die Durchgangsbohrung 37 innerhalb des Unterteils 32, so dass das Unterteil 32, das über Bolzen 34 mit dem Grundkörper 30 verschraubt ist, schwenkbar an dem Joch 6 gelagert ist. Die Schwenkachse 7 stellt dabei die Drehachse dar.

Oberhalb der Durchgangsbohrungen 67, also in proximaler Richtung, sind weitere Lagerbohrungen 65 eingebracht, bei denen im rechten Träger 62 an der Außenseite eine Innenverzahnung 63 ausgebildet ist. Innerhalb der Lagerbohrungen 65 sind Lagerringe 50, die ebenfalls als Gleitlager ausgebildet sind, angeordnet und nehmen Lagerscheiben 52 einer exzentrisch gelagerten Welle 51 auf. Die Welle 51 ist in einer Langlochführung 33, die als Durchgangsschlitz ausgebildet ist, in dem Grundkörper 30 geführt und bewirkt bei einer Verdrehung innerhalb der Lagerscheiben 50 eine Verschwenkung des Anschlussmittels 3 um die Schwenkachse 7. Wird die Welle 51 beispielsweise von einer Ausgangsposition in Richtung auf das hintere Ende des Prothesenfußes verdreht, verkippt das Anschlussmittel 3 relativ nach hinten, so dass eine Plantarflexion des Fußteils 2 erfolgt, wird die Welle 51 in Richtung der Fußspitze verdreht, bewirkt dies eine Dorsalflexion, das heißt, dass die Fußspitze in Richtung des Prothesenkniegelenkes angehoben wird.

An der im Ausführungsbeispiel rechten Lagerscheibe 52 ist eine Außenverzahnung 53 ausgebildet, die korrespondierend zu der Innenverzahnung 63 an dem Träger 62 hergestellt ist, so dass bei einem Ineinandergreifen der Verzahnungen 53, 63 eine Verdrehung der Lagerscheiben 52 in den Lagerbohrungen 65 gesperrt wird. Dadurch kann die Welle 51 nicht bewegt werden und das Anschlussmittel 3 ist in der eingestellten Position fixiert. Zum Lösen der Verrieglung oder Sperreinrichtung, die durch die Verzahnungen 53, 63 gebildet wird, wird auf die linke Lagerscheibe 52 gedrückt, so dass sowohl die Welle 51 als auch die rechte Lagerscheibe 52 in Richtung auf die Abdeckkappe 54 verlagert wird. Dadurch wird die Außenverzahnung 53, die als Formschlusselemente in die Innenverzahnung 63 wirken, außer Eingriff gebracht und ausgerückt, so dass eine freie Verdrehbarkeit der Lagerscheiben 52 innerhalb der Lagerbohrungen 65 vorgenommen werden kann.

In der Figur 8 ist ebenfalls ein Positionssensor 9 zu erkennen, der die Position des Fußteils 2, das fest verschraubt mit dem Joch 6 verbunden ist, relativ zu dem Anschlussmittel 3 detektiert. Der Positionssensor 9 kann entweder als Lagesensor ausgebildet sind, der die Orientierung de Fußteils im Raum während eines Einstellmodus detektiert, oder als Relativsensor, der ausgehend von einer Ausgangsstellung die Relativposition des Fußteils 2 über die Relativposition des Joches 6 zu dem Anschlussmittel 3 detektiert.

An dem Sensor 9 ist zudem ein einstellbarer Signalgeber 91 angeordnet, über den eingestellt werden kann, wann ein Positionssignal des Sensors 9 ausgegeben wird, um eine Vorauswahl der auszugebenden Signale zu bewirken.

Der Sensor 9 ist vorteilhafterweise als ein Hallsensor ausgebildet, bei dem entweder ein Magnet relativ zu einer Vielzahl von Spulen oder viele Magnete relativ zu einer Spule aufgrund der Drehung der Welle 51 oder der Lagerscheiben 52 relativ zu dem Sensor 9 verlagert werden. Aufgrund des Hall-Effektes wird ein Signal generiert, das ausgewertet wird und auf dessen Grundlage eine Signalausgabe durch die Ausgabeeinrichtung 10 erfolgt.

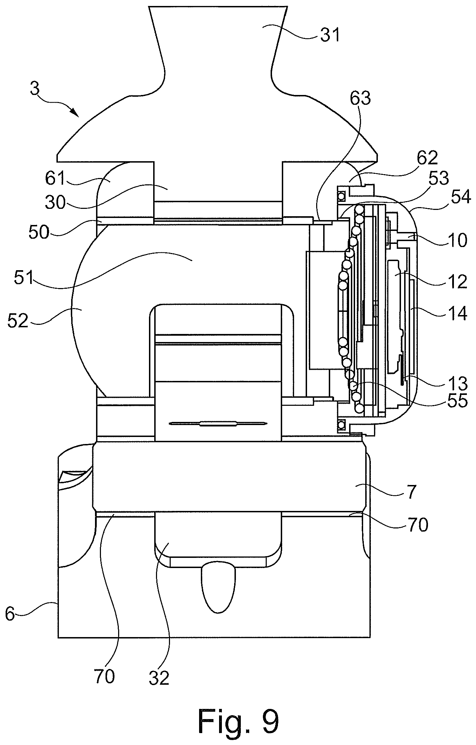

Figur 9 zeigt eine Schnittdarstellung einer Ausführungsform die im Wesentlichen der der Figur 8 entspricht. Der Positionssensor ist nicht dargestellt. In der Figur 9 ist das Anschlussmittel 3 in einem verriegelten Zustand gezeigt. Das Joch 6 mit den beiden im Wesentlichen parallel zueinander ausgerichteten Trägern 61, 62 nimmt zwischen den beiden Trägern 61, 62 das Anschlussmittel 3 und insbesondere den Grundkörper 30 und das Unterteil 32 auf. Die Schwenkachse 7 ist über die Lager 70 schwenkbar an dem Joch 6 gelagert. Innerhalb der Langlochführung in dem Grundkörper 30 ist die exzentrische Welle 51 gelagert. Die beiden Lagerscheiben 52 sind in den Lagerschalen 50 in den Lagerbohrungen 65 drehbar gelagert. Die Außenverzahnung 53 an der rechten Lagerscheibe 52 greift in die Innenverzahnung 63 am rechten Träger 62 ein und sperrt somit eine Verschwenkung des Anschlussmittels 3 um die Schwenkachse 7.

Innerhalb der Abdeckkappe 54 ist eine Druckfeder 55 gelagert, die die Lagerscheibe 52 mit der Außenverzahnung 53 in Richtung auf die Innenverzahnung 63 drückt.

Dadurch wird die formschlüssige Verriegelung der Welle 51 relativ zu dem Joch 6 sichergestellt. Zum Entsperren der Verriegelung wird eine Druckkraft auf die der Abdeckkappe 54 abgewandten Seite auf die Lagerscheibe 52 aufgebracht, so dass die Welle 51 nach rechts verschoben und die Verzahnungen 53, 63 außer Eingriff gebracht werden.

Innerhalb der Abdeckkappe 54 ist eine Steuerungseinrichtung 12 angeordnet, die ausgehend von dem empfangenen Positionssignal des Positionssensors 9 eine Ausgabeeinrichtung 10 für ein optisches und/oder akustisches und/oder haptisches Signal aktiviert. Die Ausgabeeinrichtung 10 kann beispielsweise eine Absatzhöhenstufe durch ein rhythmisches Signal anzeigen, ebenso ist es möglich, eine Sprachausgabe vorzusehen oder über ein Bestätigungssignal das Erreichen einer voreingestellten Sollposition zu melden. Die Sollposition kann durch die Steuerungseinrichtung 12 festgelegt werden, beispielsweise indem ein Detektor 19, beispielsweise ein RFID-Empfänger, ein Signal einer Absatzhöhenmarkierung 11 empfängt und einer entsprechenden Winkelstellung des Fußteils 2 relativ zu dem Anschlussmittel 3 als Sollposition festlegt. Wird diese Sollposition bei der Verstellung des Fußteils 2 relativ zu dem Anschlussmittel 3 erreicht, ertönt ein Bestätigungssignal oder wird ein anderes Bestätigungssignal ausgegeben. Auch ist es möglich, dass die Absatzhöhenmarkierung auf einer anderen Art und Weise der Steuerungseinrichtung 12 übermittelt wird, beispielsweise manuell durch Verdrehung einer Stelleinrichtung, durch wiederholtes Drücken oder Betätigen eines Schalters, um die entsprechende Absatzhöhenstufe einzugeben, durch eine Spracheingabe, durcheine Fernbedienung oder auf eine andere Art und Weise. Auch können bestimmte Schalter oder Mikroschalter manuell aktiviert werden, so dass bei Vorliegen einer entsprechenden Winkelstellung ein Bestätigungssignal ausgegeben oder ein Warnsignal, das eine nicht korrekte Absatzhöheneinstellung anzeigt, unterbrochen wird.

Der Steuerungseinrichtung 12 ist ein Sender 13 zugeordnet, über den es möglich ist, die Signale des nicht dargestellten Sensors 9 an eine entfernte Ausgabeeinrichtung 10 zu übermitteln, beispielsweise ein Mobiltelefon, ein Computer oder dergleichen. Der Steuerungseinrichtung 12 ist zudem eine Bestätigungseinrichtung 14 zugeordnet, die im dargestellten Ausführungsbeispiel als ein Bestätigungsknopf in der Abdeckkappe 54 ausgebildet ist. Der Bestätigungsknopf 14 dient dazu, bei der Zuordnung der jeweiligen Absatzhöhe zu dem Signal eine Bestätigung durchzuführen. Wird die Absatzhöhe beispielsweise chronologisch eingestellt, wird der ersten eingestellten Absatzhöhe ein erstes Signal zugeordnet, indem die eingestellte Absatzhöhe über den Bestätigungsknopf 14 der Steuereinrichtung 12 bestätigt wird. Die Steuereinrichtung 12 ordnet dieser Absatzhöhe ein erstes Signal zu. Wird eine zweite Absatzhöhe eingestellt und der Betätigungsknopf 14 erneut betätigt, wird dieser Absatzhöhe das zweite Ausgabesignal zugeordnet, unabhängig, ob die zweite Absatzhöhe größer oder kleiner als die erste Absatzhöhe ist. Wird eine Absatzhöheneinstellung gelöscht, wird diese frei und kann erneut vergeben werden, auch hier ist es nicht wesentlich, die Absatzhöhen in einer bestimmten Reihenfolge einzuspeichern oder zu ordnen. Nach dem Einstellen und Bestätigen der jeweiligen Absatzhöhe und der Zuordnung des jeweiligen Signals kann der Prothesenfuß eingesetzt werden. Wird ein Schuh gewechselt, wird die Absatzhöhe verstellt, indem die Verstelleinrichtung 5 betätigt wird. Dazu werden die Verzahnungen 53, 63 durch Verschieben entgegen der Druckkraft der Fehler 55 außer Eingriff gebracht und das Fußteil 2 relativ zu dem Anschlussmittel 3 verkippt, bis ein Signal ausgegeben wird. Das Signal kann über die Ausgabeeinrichtung 10 in Gestalt eines Lichtsignals und über einen akustischen Signalgeber an den Nutzer ausgegeben werden. Wird die gewünschte Stellung des Fußteils 2 relativ zu dem Anschlussmittel 3 erreicht, werden die Verzahnungen 53, 63 verrastet. Sofern die Verrastung erfolgt ist, kann ein zusätzliches Signal ausgegeben werden, das die Einsatzbereitschaft des Prothesenfußes signalisiert.

Neben der beschriebenen aktiven Signalausgabe ist es möglich, keine Energiequellen für die Signalausgabe bereitzustellen, sondern die Signalausgabe durch die Verstellung selbst zu bewirken. Hierzu können sogenannte Rattermarken eingesetzt werden, die die Verstellung des Fußteils 2 relativ zu dem oberen Anschlussmittel 3 taktil und akustisch ausgeben. Zusätzlich zu der Rasterung ist es möglich, dass ein akustisches Ausgabeelement vorgesehen ist, das beispielsweise durch eine überspringende Feder ein akustisches Signal aussendet, wenn eine Absatzhöhenstufe erreicht oder überschritten ist. Zusätzlich kann eine optische Anzeige der Verstellung zugeordnet sein.

Mit dem Prothesenfuß ist es möglich, eine Einstellhilfe für den Prothesenfuß bereitzustellen, um unterschiedliche Absatzhöhen reproduzierbar bei einem Gesamtprothesenaufbau, der von einem Orthopädietechniker eingestellt worden ist, anzupassen. Über die Steuerungseinrichtung 12 ist es möglich, bestimmte Absatzhöhenabstufungen vorab auszuwählen, so dass beispielsweise nach Sichtung der zur Verfügung stehenden Schuhe mit unterschiedlichen Absatzhöhen eine Einordnung durch den Orthopädietechniker erfolgt. Stehen beispielsweise vier unterschiedliche Absatzhöhen zur Verfügung, kann für jede Absatzhöhe die optimale Winkelstellung des Fußteils 2 relativ zu dem Anschlussmittel 3 einprogrammiert werden. Das Einprogrammieren in die Steuerungseinrichtung 12 kann manuell über Drucktaster, über Drehschalter oder auch über eine Drahtlosverbindung erfolgen. Dadurch wird durch den Orthopädietechniker eine Filterung der Vielzahl möglicher Absatzhöheneinstellungen auf verbleibende Einstellungen, die für den Patienten relevant sind, vorgenommen. Die Signalausgabe für eine einzustellende Absatzhöhe erfolgt dann patientenangepasst, so dass nur noch die vier ausgewählten Absatzhöhen zur Auswahl stehen und nicht sämtliche Absatzhöhenabstufungen, die sehr fein abgestuft vorliegen können, mitgezählt oder wahrgenommen werden müssen. Somit erfolgt die Signalisierung der relevanten Absatzhöheneinstellungen nach einer Vorauswahl durch den Orthopädietechniker, so dass der Patient nicht durch nicht relevante Stellungssignale irritiert wird. Es besteht auch die Möglichkeit, dass die vom Orthopädietechniker ausgewählten Stellungen mit einem anderen Signal versehen werden als die übrigen Raststellungen, beispielsweise durch eine Abweichung in Klang, Lautstärke, Lichtfarbe oder dergleichen.

Die Absatzhöhenmarkierung 11 kann auch über ein Sprachkommando eingegeben werden, so dass ein bestimmter Schuh mit einer entsprechenden Markierung dem Prothesenfuß angesagt wird und über eine Spracherkennung die jeweils zugeordnete Absatzhöhe mit einem entsprechenden Signal versehen wird.

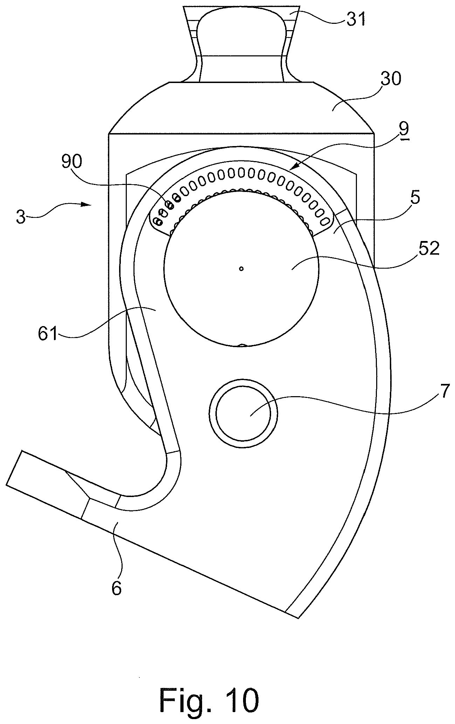

In der Figur 10 ist in einer Seitenansicht eine Variante der Erfindung gezeigt, die in ihrem Grundaufbau dem der Figur 6 entspricht. Der Positionssensor 9 ist in dem dargestellten Ausführungsbeispiel als mechanischer Sensor ausgebildet, in dem sogenannte Dip-Schalter 90 integriert sind, die sich über einen Kreisbogen entlang eines Verschwenkweges des proximalen Anschlussmittels 3 relativ zu dem Joch 6 erstrecken. Durch Verschieben der Dip-Schalter 90 werden Rasteinrichtungen gesperrt oder freigegeben, Kontakte gesperrt oder freigegeben oder beides ausgeführt, so dass das Erreichen einer bestimmten Position des Grundkörpers 30 relativ zu dem Joch 6 oder der Verstelleinrichtung 5 angezeigt, gespürt oder gehört wird und/oder angezeigt oder ausgegeben werden kann. Im dargestellten Ausführungsbeispiel sind in den linken Ausnehmungen verschiedene Stellungen des jeweiligen Dip-Schalters 90 gezeigt.

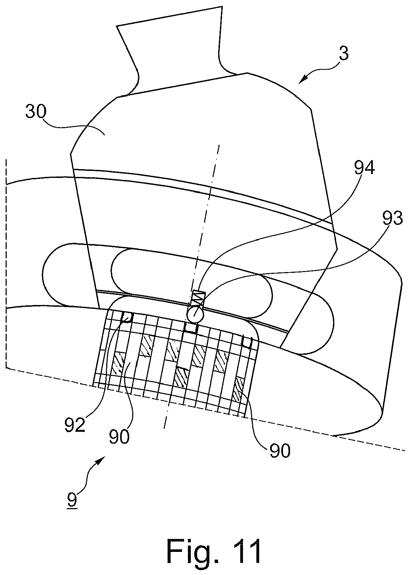

Figur 11 zeigt eine schematische Darstellung der Funktionsweise und des Aufbaus der mechanischen Positionssensoranordnung gemäß Figur 10 in einer Schnittdarstellung. Das proximale Anschlussmittel 3 mit dem Grundkörper 30 ist wiederum verschwenkbar um die nicht dargestellte Schwenkachse 7 an dem Joch gelagert. Die Dip-Schalter 90 des mechanischen Positionssensors 9 sind in gleichmäßigen Abständen zueinander fächerförmig angeordnet, wobei insgesamt zehn Dip-Schalter 90 im dargestellten Ausführungsbeispiel vorgesehen sind. Die beiden jeweils äußeren Dip-Schalter 90 sowie der mittlere Dip-Schalter 90 sind in einer zurückgezogenen, in Richtung auf die Schwenkachse 6 orientierten Freigabestellung angeordnet, die übrigen sind in einer Sperrstellung orientiert. In der Freigabestellung werden Rastausnehmungen 92 von den Dip-Schaltern 90 freigegeben, in die ein Rastelement 93 in Gestalt einer Kugel einrasten kann. Die Kugel 93 ist in Richtung auf die Rastausnehmungen 92 durch eine Feder 94 vorgespannt, so dass bei einer Verstellung des Anschlussmittels 3 die Kugel 93 in der jeweiligen freigegebenen Rastausnehmung 92 einrastet. Durch die gleichmäßige, fächerförmige Anordnung der Dip-Schalter 90 wird über den gesamten Verschwenkweg des Anschlussmittels 3 in gleichmäßigen Winkelabständen eine Anzeige in akustischer und taktiler Art und Weise möglich. Die jeweils beiden äußeren Dip-Schalter 90 und die dazugehörigen Rastausnehmungen 92 stellen die Endpunkte der Verschwenkbarkeit dar. In der dargestellten Ausführungsform ist das proximale Anschlussmittel 3 in einer mittleren Stellung positioniert, von der aus eine Verschwenkung nach gegebenenfalls einer Entriegelung in die jeweilige Verschwenkrichtung erfolgen kann.

Durch das Einrasten der Kugel 93 in die Rastausnehmung 92 ist es auch möglich, einen elektronischen Kontakt zu schließen, so dass ein Signal über das Erreichen einer bestimmten Winkelposition auf elektronischem Wege ausgegeben werden kann, beispielsweise über einen Computer, ein Mobiltelefon oder eine speziell dafür ausgerüstete Anzeigeeinrichtung. Durch den Kontakt kann auch ein akustisches Signal ausgegeben werden, dass über das mechanische Einrastgeräusch der Kugel 93 in der Rastausnehmung 92 hinausgeht und separat erzeugt wird, beispielsweise über einen Signalgenerator in Gestalt eines Lautsprechers oder einen anderen Schallerzeugungseinrichtung, die bei Schließen des Kontaktes aktiviert wird und aktiv ein Signal, das von dem Einrastgeräusch abweicht, erzeugt.

Bei geschlossenen Dip-Schaltern 90 rollt die Kugel 93 auf der Oberfläche der Dip-Schalter, die in der Sperrstellung die Rastausnehmungen 92 verschließen, ab, so dass keine haptische und/oder akustische Rückmeldung an den Nutzer erfolgt. Neben einer gleich beabstandeten Anordnung der Dip-Schalter 90 nebeneinander ist es möglich, diese auch in ungleichmäßigen Abständen voneinander anzuordnen, um bei Vorzugspositionen eine präzise Einstellbarkeit zu ermöglichen, so dass auch Zwischenpositionen eingenommen werden können.

Neben der in der Figur 10 gezeigten Anordnung von Dip-Schaltern 90 nur an der oberen Hälfte um die Lagerscheibe 51 herum ist es auch möglich, Dip-Schalter zusätzlich auf der gegenüberliegenden, unteren Seite anzuordnen, wobei die Dip-Schalter an der unteren Seite dort um die Hälfte der Teilung versetzt sind, so dass eine halbierte Stufung der Winkeleinstellbarkeit möglich ist. Hierdurch können feinere Einstellbarkeiten bei gleichzeitig ausreichender Größe der Dip-Schalter 90 erreicht werden, so dass die Handhabbarkeit der Dip-Schalter 90 weiterhin gewährleistet wird. Die Dip-Schalter können als Schiebeschalter ausgebildet sein, die in Führungen längsverschieblich gelagert sind. Durch die Ausgestaltung des Formschlusselementes 93 als Kugel ist es möglich, gegen einen gewissen Widerstand durch die Feder 94 ein Abrollen auf der Oberfläche der Dip-Schalter in der Schließstellung zu ermöglichen und die Kugel 93 aus der Ausnehmung 92 herauszubewegen.

tierten Freigabestellung angeordnet, die übrigen sind in einer Sperrstellung orientiert. In der Freigabestellung werden Rastausnehmungen 92 von den Dip-Schaltern 90 freigegeben, in die ein Rastelement 93 in Gestalt einer Kugel einrasten kann. Die Kugel 93 ist in Richtung auf die Rastausnehmungen 92 durch eine Feder 94 vorgespannt, so dass bei einer Verstellung des Anschlussmittels 3 die Kugel 93 in der jeweiligen freigegebenen Rastausnehmung 92 einrastet. Durch die gleichmäßige, fächerförmige Anordnung der Dip-Schalter 90 wird über den gesamten Verschwenkweg des Anschlussmittels 3 in gleichmäßigen Winkelabständen eine Anzeige in akustischer und taktiler Art und Weise möglich. Die jeweils beiden äußeren Dip-Schalter 90 und die dazugehörigen Rastausnehmungen 92 stellen die Endpunkte der Verschwenkbarkeit dar. In der dargestellten Ausführungsform ist das proximale Anschlussmittel 3 in einer mittleren Stellung positioniert, von der aus eine Verschwenkung nach gegebenenfalls einer Entriegelung in die jeweilige Verschwenkrichtung erfolgen kann.

Durch das Einrasten der Kugel 93 in die Rastausnehmung 92 ist es auch möglich, einen elektronischen Kontakt zu schließen, so dass ein Signal über das Erreichen einer bestimmten Winkelposition auf elektronischem Wege ausgegeben werden kann, beispielsweise über einen Computer, ein Mobiltelefon oder eine speziell dafür ausgerüstete Anzeigeeinrichtung. Durch den Kontakt kann auch ein akustisches Signal ausgegeben werden, dass über das mechanische Einrastgeräusch der Kugel 93 in der Rastausnehmung 92 hinausgeht und separat erzeugt wird, beispielsweise über einen Signalgenerator in Gestalt eines Lautsprechers oder einen anderen Schallerzeugungseinrichtung, die bei Schließen des Kontaktes aktiviert wird und aktiv ein Signal, das von dem Einrastgeräusch abweicht, erzeugt.

Bei geschlossenen Dip-Schaltern 90 rollt die Kugel 93 auf der Oberfläche der Dip-Schalter, die in der Sperrstellung die Rastausnehmungen 92 verschließen, ab, so dass keine haptische und/oder akustische Rückmeldung an den Nutzer erfolgt. Neben einer gleich beabstandeten Anordnung der Dip-Schalter 90 nebeneinander ist es möglich, diese auch in ungleichmäßigen Abständen voneinander anzuordnen, um bei Vorzugspositionen eine präzise Einstellbarkeit zu ermöglichen, so dass auch Zwischenpositionen eingenommen werden können.

Neben der in der Figur 10 gezeigten Anordnung von Dip-Schaltern 90 nur an der oberen Hälfte um die Lagerscheibe 51 herum ist es auch möglich, Dip-Schalter zusätzlich auf der gegenüberliegenden, unteren Seite anzuordnen, wobei die Dip-Schalter an der unteren Seite dort um die Hälfte der Teilung versetzt sind, so dass eine halbierte Stufung der Winkeleinstellbarkeit möglich ist. Hierdurch können feinere Einstellbarkeiten bei gleichzeitig ausreichender Größe der Dip-Schalter 90 erreicht werden, so dass die Handhabbarkeit der Dip-Schalter 90 weiterhin gewährleistet wird. Die Dip-Schalter können als Schiebeschalter ausgebildet sein, die in Führungen längsverschieblich gelagert sind. Durch die Ausgestaltung des Formschlusselementes 93 als Kugel ist es möglich, gegen einen gewissen Widerstand durch die Feder 94 ein Abrollen auf der Oberfläche der Dip-Schalter in der Schließstellung zu ermöglichen und die Kugel 93 aus der Ausnehmung 92 herauszubewegen.

## Patentansprüche

1. Prothesenfuß mit einem Fußteil (2), einem proximalen Anschlussmittel (3), das schwenkbar mit dem Fußteil (2) verbunden ist und einer Verstelleinrichtung (5), mit der das Fußteil (2) relativ zu dem Anschlussmittel (3) verstellbar ist, **dadurch gekennzeichnet, dass** der Verstelleinrichtung (5) zumindest ein Positionssensor (9) zugeordnet ist, der mit einem Signalerzeugungselement (10) gekoppelt ist, das in Abhängigkeit von dem Signal des zumindest einen Positionssensors (9) ein Signal über das Erreichen einer Position des Fußteils (2) ausgibt.

2. Prothesenfuß nach Anspruch 1, **dadurch gekennzeichnet, dass** das Signalerzeugungselement (10) als Ausgabeeinrichtung für optische, taktile und/oder akustische Signale ausgebildet ist.

3. Prothesenfuß nach Anspruch 1 oder 2, **dadurch gekennzeichnet, dass** die Verstelleinrichtung (5) Rastelemente (53, 63) aufweist, über die die Verstellung des Fußteils (2) relativ zu dem Anschlussmittel (3) in diskreten Schritten erfolgt.

4. Prothesenfuß nach einem der voranstehenden Ansprüche, **dadurch gekennzeichnet, dass** der Positionssensor (9) als Lagesensor, Relativwinkelsensor, Inertialwinkelsensor und/oder Schalter ausgebildet ist.

5. Prothesenfuß nach einem der voranstehenden Ansprüche, **dadurch gekennzeichnet, dass** dem Positionssensor (9) ein einstellbarer oder programmierbarer Signalgeber (91) zugeordnet ist.

6. Prothesenfuß nach einem der voranstehenden Ansprüche, **dadurch gekennzeichnet, dass** der Verstelleinrichtung (5) zumindest ein Empfänger oder ein Detektor (19) von Schuhidentifikationsdaten zugeordnet ist.

7. Prothesenfuß nach Anspruch 6, **dadurch gekennzeichnet, dass** der Empfänger oder Detektor mit dem Positionssensor (9) und/oder dem Signalerzeugungselement (10) gekoppelt ist.

8. Prothesenfuß nach einem der voranstehenden Ansprüche, **dadurch gekennzeichnet, dass** der Verstelleinrichtung (5) ein Antrieb zugeordnet ist.

9. Prothesenfuß nach einem der voranstehenden Ansprüche, **dadurch gekennzeichnet, dass** die Verstelleinrichtung (5) über eine exzentrisch gelagerte Welle (51) mit dem Anschlussmittel (3) gekoppelt ist.

10. Prothesenfuß nach Anspruch 9, **dadurch gekennzeichnet, dass** die Welle (51) in einer Langlochführung (33) geführt ist.

11. Prothesenfuß nach einem der voranstehenden Ansprüche, **dadurch gekennzeichnet, dass** das Anschlussmittel (3) eine deaktivierbare Sperreinrichtung (53, 63) aufweist, die eine Verdrehung des Fußteils (2) relativ zu dem Anschlussmittel (3) sperrt.

12. Prothesenfuß nach Anspruch 11, **dadurch gekennzeichnet, dass** die Sperreinrichtung (53, 63) ausrückbare Formschlusselemente (53, 63) aufweist.

13. Prothesenfuß nach einem der voranstehenden Ansprüche, **dadurch gekennzeichnet, dass** das Signalerzeugungselement (10) mit einem Sender (13) gekoppelt ist.

14. Prothesenfuß nach einem der voranstehenden Ansprüche, **dadurch gekennzeichnet, dass** das Signalerzeugungselement (10) als aktives Signalerzeugungselement (10) ausgebildet ist.

15. System aus einem Prothesenfuß nach einem der voranstehenden Ansprüche mit einem Schuh (1), der eine auslesbare Absatzhöhenmarkierung (11) aufweist.

16. System nach Anspruch 15, **dadurch gekennzeichnet, dass** die Absatzhöhenmarkierung (11) in einem RFID-Chip, einem Transponder, einem optisch oder einem opto-elektronisch auslesbaren Code abgelegt ist.

17. Verfahren zum Anpassen der Absatzhöhe eines Prothesenfußes nach einem der voranstehenden Ansprüche an einem Schuh (1), dem eine Absatzhöhenmarkierung (11) zugeordnet ist, wobei die Absatzhöhenmarkierung (11) an eine Steuerungseinrichtung (12) übermittelt und mit erfassten Positionsdaten des Positionssensors (9) verglichen und ein Bestätigungssignal ausgegeben wird, wenn das der jeweiligen Absatzhöhenmarkierung (11) vorab zugeordnete Positionssignal mit dem erfassten Positionssignal übereinstimmt.

18. Verfahren nach Anspruch 17, **dadurch gekennzeichnet, dass** die Absatzhöhenmarkierung (11) automatisch ausgelesen und der Steuerungseinrichtung (12) übermittelt wird.

19. Verfahren nach Anspruch 17 oder 18, **dadurch gekennzeichnet, dass** das Anschlussmittel (3) relativ zu dem Fußteil (2) motorisch verlagert wird.

## Claims

1. A prosthetic foot having a foot part (2), a proximal connection means (3) which is connected tiltably to the foot part (2), and an adjustment device (5) with which the foot part (2) can be adjusted relative to the connection means (3), **characterized in that** the adjustment device (5) is assigned at least one position sensor (9), which is coupled to a signal generating element (10), which outputs a signal about reaching a position of the foot part (10) as a function of the signal of the at least one position sensor (9).

2. The prosthetic foot as claimed in claim 1, **characterized in that** the signal generating element (10) is configured as an output device for optical, tactile and/or acoustic signals.

3. The prosthetic foot as claimed in claim 1 or 2, **characterized in that** the adjustment device (5) comprises latch elements (53, 63), by means of which the adjustment of the foot part (2) relative to the connection means (3) takes place in discrete steps.

4. The prosthetic foot as claimed in one of the preceding claims, **characterized in that** the position sensor (9) is configured as a location sensor, relative angle sensor, inertial angle sensor and/or switch.

5. The prosthetic foot as claimed in one of the preceding claims, **characterized in that** the position sensor (9) is assigned an adjustable or programmable signal generator (91).

6. The prosthetic foot as claimed in one of the preceding claims, **characterized in that** the adjustment device (5) is assigned at least one receiver or a detector (19) of shoe identification data.

7. The prosthetic foot as claimed in claim 6, **characterized in that** the receiver or detector is coupled to the position sensor (9) and/or to the signal generating element (10).

8. The prosthetic foot as claimed in one of the preceding claims, **characterized in that** the adjustment device (5) is assigned a drive.

9. The prosthetic foot as claimed in one of the preceding claims, **characterized in that** the adjustment device (5) is coupled to the connection means (3) by means of an eccentrically mounted shaft (51).

10. The prosthetic foot as claimed in claim 9, **characterized in that** the shaft (51) is guided in a longitudinal hole guide (33).

11. The prosthetic foot as claimed in one of the preceding claims, **characterized in that** the connection means (3) comprise a deactivatable blocking device (53, 63), which blocks rotation of the foot part (2) relative to the connection means (3).

12. The prosthetic foot as claimed in claim 11, **characterized in that** the blocking device (53, 63) comprises retractable form-fit elements (53, 63).

13. The prosthetic foot as claimed in one of the preceding claims, **characterized in that** the signal generating element (10) is coupled to a transmitter (13).

14. The prosthetic foot as claimed in one of the preceding claims, **characterized in that** the signal generating element (10) is configured as an active signal generating element (10).

15. A system consisting of a prosthetic foot as claimed in one of the preceding claims with a shoe (1), which has a readable heel height marking (11).

16. The system as claimed in claim 15, **characterized in that** the heel height marking (11) is stored in an RFID chip, a transponder, or an optically or optoelectronically readable code.

17. A method for adapting the heel height of a prosthetic foot as claimed in one of the preceding claims to a shoe (1), which is assigned a heel height marking (11), wherein the heel height marking (11) is transmitted to a control device (12) and compared with detected position data of the position sensor (9), and a confirmation signal is emitted when the position signal assigned beforehand to the respective heel height marking (11) matches with the detected position signal.

18. The method as claimed in claim 17, **characterized in that** the heel height marking (11) is automatically read and transmitted to the control device (12).

19. The method as claimed in claim 17 or 18, **characterized in that** the connection means (3) is displaced in a motorized fashion relative to the foot part (2).

## Revendications

1. Prothèse de pied comportant une partie pied (2), un moyen de raccordement proximal (3) qui est relié mobile en basculement à la partie pied (2), et un dispositif de réglage (5) permettant de régler la partie pied (2) par rapport au moyen de raccordement (3), **caractérisée en ce que** au moins un capteur de position (9) est associé au dispositif de réglage (5), qui est couplé à un élément de génération de signal (10) qui émet un signal sur l'atteinte d'une position de la partie pied (2) en fonction du signal dudit au moins un capteur de position (9).

2. Prothèse de pied selon la revendication 1, **caractérisée en ce que** l'élément de génération de signal (10) est réalisé sous la forme d'un moyen d'émission pour des signaux optiques, tactiles et/ou acoustiques.

3. Prothèse de pied selon la revendication 1 ou 2, **caractérisée en ce que** le dispositif de réglage (5) comprend des éléments d'enclenchement (53, 63) par lesquels s'effectue le réglage de la partie pied (2) par rapport au moyen de raccordement (3) en étapes discrètes.

4. Prothèse de pied selon l'une des revendications précédentes, **caractérisée en ce que** le capteur de position (9) est réalisé sous forme de capteur de positionnement, de capteur d'angle relatif, de capteur d'angle inertiel et/ou sous forme de commutateur.

5. Prothèse de pied selon l'une des revendications précédentes, **caractérisée en ce qu'**un émetteur de signal réglable ou programmable (91) est associé au capteur de position (9).

6. Prothèse de pied selon l'une des revendications précédentes, **caractérisée en ce qu'**au moins un récepteur ou un détecteur (19) de données d'identification de chaussures est associé au dispositif de réglage (5).

7. Prothèse de pied selon la revendication 6, **caractérisée en ce que** le récepteur ou le détecteur est couplé au capteur de position (9) et/ou à l'élément de génération de signal (10).

8. Prothèse de pied selon l'une des revendications précédentes, **caractérisée en ce qu'**un entraînement est associé au dispositif de réglage (5).

9. Prothèse de pied selon l'une des revendications précédentes, **caractérisée en ce que** le dispositif de réglage (5) est couplé au moyen de raccordement (3) par un arbre (51) monté de façon excentrique.

10. Prothèse de pied selon la revendication 9, **caractérisée en ce que** l'arbre (51) est guidé dans un guidage à trou oblong (33).

11. Prothèse de pied selon l'une des revendications précédentes, **caractérisée en ce que** le moyen de raccordement (3) présente un dispositif de blocage désactivable (53, 63) qui bloque une rotation de la partie pied (2) par rapport au moyen de raccordement (3).

12. Prothèse de pied selon la revendication 11, **caractérisée en ce que** le dispositif de blocage (53, 63) comprend des éléments de coopération de formes (53, 63) escamotables.

13. Prothèse de pied selon l'une des revendications précédentes, **caractérisée en ce que** l'élément de génération de signal (10) est couplé à un émetteur (13).

14. Prothèse de pied selon l'une des revendications précédentes, **caractérisée en ce que** l'élément de génération de signal (10) est réalisé sous la forme d'un élément de génération de signal (10) actif.

15. Système constitué par une prothèse de pied selon l'une des revendications précédentes avec une chaussure (1) qui comprend un marquage de hauteur de talon (11) lisible.

16. Système selon la revendication 15, **caractérisé en ce que** le marquage de hauteur de talon (11) est stocké dans une puce RFID, un transpondeur, un code lisible par voie optique ou par voie optoélectronique.

17. Procédé pour adapter la hauteur de talon d'une prothèse de pied selon l'une des revendications précédentes sur une chaussure (1) à laquelle est associé un marquage de hauteur de talon (11),
dans lequel le marquage de hauteur de talon (11) est transmis à un dispositif de commande (12) et est comparé à des données de position acquises du capteur de position (9), et un signal de confirmation est émis lorsque le signal de position associé auparavant au marquage de hauteur de talon (11) respectif coïncide avec le signal de position acquis.

18. Procédé selon la revendication 17, **caractérisé en ce que** le marquage de hauteur de talon (11) est lu automatiquement et transmis au dispositif de commande (12).

19. Procédé selon la revendication 17 ou 18, **caractérisé en ce que** le moyen de raccordement (3) est déplacé par rapport à la partie pied (2) par voie motorisée.
